# EUROPEAN PATENT SPECIFICATION

(11) **EP 2 609 915 B1**
(45) Date of publication and mention of the grant of the patent: **27.04.2016**
(21) Application number: 12008487.6
(22) Date of filing: 20.12.2012
(51) Int. Cl.: A61K 31/19, A61K 31/194, A61K 33/00, A61K 33/06, A61K 33/14, A61K 33/42, A61P 13/00, A61K 31/198

(54) **Dialysis and substitution fluid**
Dialyse- und Substitutionsfluid
Dialyse et fluide de substitution

(30) Priority: 28.12.2011 CZ 201125404 U
(43) Date of publication of application: 03.07.2013
(73) Proprietor: GML Health Care s.r.o., 155 00 Praha 5 (CZ)
(72) Inventor: Balík, Martin, 155 00 Praha (CZ)
(74) Representative: Reichel, Pavel

(56) References cited:
- MORGERA STANISLAO ET AL: "Regional citrate anticoagulation in continuous hemodialysis - Acid-base and electrolyte balance at an increased dose of dialysis", NEPHRON CLINICAL PRACTICE, vol. 101, no. 4, 2005, pages C211-C219, XP8160977, ISSN: 1660-2110
- HELEEN M OUDEMANS-VAN STRAATEN ET AL: "Clinical review: Anticoagulation for continuous renal replacement therapy - heparin or citrate?", CRITICAL CARE, vol. 15, no. 1, 25 January 2011 (2011-01-25), page 202, XP55055459, ISSN: 1364-8535, DOI: 10.1186/cc9358
- MORGERA S: "Survival benefit with regional anticoagulation with citrate in CRRT: The end of a myth?", NEPHROLOGY DIALYSIS TRANSPLANTATION 2011 OXFORD UNIVERSITY PRESS GBR, vol. 26, no. 1, 8 December 2011 (2011-12-08), pages 9-11, XP008161296, ISSN: 0931-0509
- PAMELA FALL ET AL: "Continuous Renal Replacement Therapy: Cause and Treatment of Electrolyte Complications", SEMINARS IN DIALYSIS, vol. 23, no. 6, 2010, pages 581-585, XP55059079, ISSN: 0894-0959, DOI: 10.1111/j.1525-139X.2010.00790.x
- PALSSON R ET AL: "Choice of replacement solution and anticoagulant in continuous venovenous hemofiltration", CLINICAL NEPHROLOGY 200601 DE, vol. 65, no. 1, January 2006 (2006-01), pages 34-42, XP008161301, ISSN: 0301-0430
- BALIK M ET AL: "Prostacyclin versus citrate in continuous haemodiafiltration: An observational study in patients with high risk of bleeding", BLOOD PURIFICATION, KARGER, BASEL, CH, vol. 23, no. 4, 23 August 2005 (2005-08-23), pages 325-329, XP008160619, ISSN: 0253-5068, DOI: 10.1159/000087770 [retrieved on 2005-08-23]

## Description

### Field of the invention

The invention refers to dialysis and substitution fluid for renal replacement therapy with citrate anticoagulation applied in critically ill patients in an intensive care units.

The present invention is defined in the appended claims. Subject matters which are not encompassed by the scope of the claims do not form part of the present invention.

### Description of the prior art

Critically ill patients hospitalized in the intensive care units require some form of continuous renal replacement therapy (CRRT) in 3 to 25% of cases according to the character of the unit. Blood flow through the extracorporeal circuit requires anticoagulation treatment because foreign surface activates coagulation cascade with subsequent loss of blood elements. This may result in a decrease of blood platelets, loss of coagulation factors, potentiation of systemic inflammation and subsequent risk of bleeding. These phenomenons may be limited with anticoagulation, i.e. application of heparin however, it's sometimes difficult titration may put a critically ill patient at risk of bleeding from multiple sites (wounds, gastrointestinal tract, invasive lines, central nervous system....)(Kleger GR, Fassler E: Can circuit lifetime be a quality indicator in continuous renal replacement therapy in the critically ill? Int J Artif Organs 2010, 33:139-146; Betjes MG, van Oosterom D, van Agteren M, van de Wetering J: Regional citrate versus heparin anticoagulation during venovenous hemofiltration in patients at low risk for bleeding: similar hemofilter survival but significantly less bleeding. J Nephrol. 2007; 20: 602-8). The citrate anticoagulation presents the best and safest option. A growing number of patients who require CRRT is on citrate anticoagulation in developed health care systems (Oudemans van Straaten HM, Bellomo R, Kellum JA: Clinical review: Anticoagulation for continuous renal replacement therapy - heparin or citrate? Crit Care 2011, 15: R202; Oudemans-van Straaten HM, Wester JP, de Pont AC, Schetz MR: Anticoagulation strategies in continuous renal replacement therapy: can the choice be evidence based? Intensive Care Med 2006, 32:188-202; Monchi M, Berghmans D, Ledoux D, et al: Citrate vs. heparin for anticoagulation in continuous venovenous hemofiltration: a prospective randomized study. Intensive Care Med 2005, 30: 260-265; Oudemans-van Straaten HM, Bosman RJ, Koopmans M, et al: Citrate anticoagulation for continuous venovenous hemofiltration. Crit Care Med 2009, 37: 545-552; Hetzel GR, Schmitz M, Wissing H, et al: Regional citrate versus systemic heparin for anticoagulation in critically ill patients on continuous venovenous haemofiltration: a prospective randomized multicentre trial. Nephrol Dial Transplant 2011, 26: 232-239; Balik M, Waldauf P, Plasil P, Pachl J: Prostacyclin versus citrate in continuous haemodiafiltration: an observational study in patients with high risk of bleeding. Blood Purif 2005, 23:325-329). A citrate modality requires application of commercially or locally available citrate solutions together with adapted dialysis and substitution fluids.

A composition of fluids designed for continuous renal replacement therapies (CRRT) in intensive care originates from fluids developed for peritoneal dialysis in the past. These fluids demand an active approach of intensivists, patient's homeostatic controls and regular corrections of fluid ion and acid-base content. The corrections are far more necessary when citrate anticoagulation is applied because of its impact on acid-base and ion homeostasis. Table 1 shows examples of the commercially available fluids. Its content is derived from plasma and the first three solutions (Baxter E2, Medisol K2 a Medisol Bi0) are primarily designed for heparin anticoagulation. The other fluids in the right part of the Table 1 (Fresenius CiCa K2, GML Citralysate K2, Prism0cal) are developed for citrate anticoagulation. A need for reduction of sodium content is obvious because citrate in infused as a sodium salt to the proximal extracorporeal circuit. This brings a risk of hypernatremia particularly with application of trisodium citrate because about 40-50% of infused citrate is eliminated on the filter and 50-60% enters patient's systemic circulation under various CRRT configurations (Mariano F, Morselli M, Bergamo D, et al: Blood and ultrafiltrate dosage of citrate as a useful and routine tool during continuous venovenous haemodiafiltration in septic shock patients. Nephrol Dial Transplant 2011, 26: 3882-3888; Balik M, Zakharchenko M, Otahal M, et al: Quantification of systemic dose of substrates for intermediate metabolism during citrate anticoagulation of continous renal replacement therapy. Blood Purif 2012, 33: 80-87; Balik M, Zakharchenko M, Leden P, et al: Bioenergetic gain of citrate anticoagulated continuous haemodiafiltration - comparative study of two algorithms. J Crit Care 2012, published on line 27.8., Epub ahead of print).

**Table 1: Overview of commercially available dialysis/substitution fluids applicable in CRRT. The costs include VAT and distribution, are calculated in Czech crowns as of December 2011 and converted to EUR (25 CZk=1 EUR). *Prism0cal requires administration only with the anticoagulation fluid Prismocitrate 10/2 in predilution (Content: citrate 10 mmol/l, citric acid 2.0 mmol/l, Na 140 mmol/l, CI 106, Osm 254 mOsm/kg).**

| Content in (mmol/l) | Baxter E2 | Medisol K2 | Medisol Bi0 | Fresenius CiCa K2 | GML Citralysate K2 | Prism0cal * |
|---|---|---|---|---|---|---|
| Na | 142 | 140 | 141 | 133 | 133 | 140 |
| K | 1.5 | 2.0 | - | 2.0 | 2.0 | - |
| Ca | 1.90 | 1.50 | 0.80 | - | - | - |
| Mg | 0.75 | 0.75 | 1.00 | 0.75 | 0.75 | 0.5 |
| Cl | 108.8 | 106.5 | 103.8 | 116.5 | 116.5 | 106 |
| P | - | - | 0.80 | - | - | - |
| Na-lactate | 40 | 40 | - | - | - | 3.0 |
| HCO₃⁻ | - | - | 40 | 20 | 20 | 32 |
| glucose | 5.05 | 6.0 | 6.7 | 5.6 | 5.6 | - |
| acetate | - | - | 4.2 | - | | - |
| Osm | 300 | 296.0 | 298.3 | | 278.0 | 282 |
| cost (EUR/5 I) | 8.40 | 12.92 | 17.64 | 19.28 | 18.00 | not found |

Table 1 shows in the last right column a composition of substitution fluid of Gambro-Hospal which requires a combination with isotonic and low citrate content (about 12 mmol/l) fluid. This is less concentrated than other fluids but is infused in significantly larger amount. A disadvantage of low concentrated citrate fluids is less efficient anticoagulation with reported almost half filter survival compared to approximately ten times more concentrated trisodium citrate (136 mmol/l) - see Palsson R, Niles JL: Regional citrate anticoagulation in continuous venovenous hemofiltration in critically ill patients with a high risk of bleeding. Kidney Int 1999, 55: 1991-7.

Nevertheless, even special fluids for citrate anticoagulation shown in Table 1 require certain minimal flow through dialysis or to reduce blood flow in the system to avoid hypernatremia and alkalosis (vide infra). The reason is dosage of citrate to the proximal blood set which is directly related to blood flow and citrate elimination on the filter which is directly related to dialysis flow (see Oudemans van Straaten HM, Bellomo R, Kellum JA: Clinical review: Anticoagulation for continuous renal replacement therapy - heparin or citrate? Crit Care 2011, 15: R202; Mariano F, Morselli M, Bergamo D, et al: Blood and ultrafiltrate dosage of citrate as a useful and routine tool during continuous venovenous haemodiafiltration in septic shock patients. Nephrol Dial Transplant 2011, 26: 3882-3888; Balik M, Zakharchenko M, Otahal M, et al: Quantification of systemic dose of substrates for intermediate metabolism during citrate anticoagulation of continous renal replacement therapy. Blood Purif 2012, 33: 80-87; Balik M, Zakharchenko M, Leden P, et al: Bioenergetic gain of citrate anticoagulated continuous haemodiafiltration - comparative study of two algorithms. J Crit Care 2012, Aug 27, Epub ahead of print; Morgera S, Scholle C, Voss G, et al: Metabolic Complications during Regional Citrate Anticoagulation in Continuous Venovenous Hemodialysis: Single-Center Experience. Nephron Clin Pract 2004, 97: 131-136; Morgera S, Haase M, Ruckert M, et al: Regional Citrate Anticoagulation in Continuous Hemodialysis - Acid-Base and Electrolyte Balance at an Increased Dose of Dialysis. Nephron Clin Pract 2005, 101: 211-219; Morgera S, Schneider M; Slowinski T, et al: A safe citrate anticoagulation protocol with variable treatment efficacy and excellent control of the acid-base status. Crit Care Med 2009, 37: 2018-2024).

With regards to the recently published large randomized controlled trials dealing with dosage of dialysis/filtration, morbidity and mortality in critically ill (see RENAL Replacement Therapy Study Investigators, Bellomo R, Cass A, Cole L, et al: Intensity of continuous renal-replacement therapy in critically ill patients. N Engl J Med 2009, 361: 1627-38; VA/NIH Acute Renal Failure Trial Network, Palevsky PM, Zhang JH, O'Connor TZ, et al: Intensity of renal support in critically ill patients with acute kidney injury. N Engl J Med 2008; 359: 7-20) current dosage is rather between 20-25 ml/kg.h than previously suggested higher dosage of 35 ml/kg.h. Another supportive rationale to reduce the dosage is the economical one. Thus, dosage of dialysis around 20-25 ml/kg.h associates with relatively slow blood flow between 90-110 ml/min to avoid inadequately high dosage of citrate. This slow blood flow may, however, reduce ultrafiltration in certain clinical scenarios (e.g. lung oedema, postdilutional continuous hemofiltration - CWH) because amount of plasmatic fluid ultrafiltrated per hour should not exceed 20% of hourly blood flow. If this happens the hemoconcentration occurs at the venous end of the filter with subsequent risk of clotting and loss of blood elements as well as loss of filter itself.

Citrate is metabolised to bicarbonate (one molecule of citrate gives rise to three molecules of bicarbonate) which may lead to metabolic alkalosis (see Oudemans van Straaten HM, Bellomo R, Kellum JA: Clinical review: Anticoagulation for continuous renal replacement therapy - heparin or citrate? Crit Care 2011, 15: R202; Oudemans-van Straaten HM, Wester JP, de Pont AC, Schetz MR: Anticoagulation strategies in continuous renal replacement therapy: can the choice be evidence based? Intensive Care Med 2006, 32:188-202). Therefore, the fluids designed for citrate anticoagulation have reduced buffer base, dosage of citrate is heavily related to blood flow and its elimination is related to adequate dialysis/substitution flow (vide supra). When bicarbonate buffer is applied the adjustment for citrate modality lies in reduction of bicarbonate content and its replacement with chloride anion.

More physiologic bicarbonate buffer has replaced lactate buffered fluids in the recent years. Interestingly, there is no evidence that patients on bicarbonate buffered fluids would do better than on the lactate buffered fluids. What is more, lactate is a great fuel for intermediate metabolism under stress conditions, sepsis and renal failure. Available papers also show its significant dosage as a buffer during various modalities of CRRT (Bollmann MD, Revelly JP, Tappy L, et al: Effect of bicarbonate and lactate buffer on glucose and lactate metabolism during haemodiafiltration in patients with multiple organ failure. Intensive Care Med 2004, 30:1103-1110; Levy B, Mansart A, Montemont C, et al: Myocardial lactate deprivation is associated with decreased cardiovascular performance, decreased myocardial energetics, and early death in endotoxic shock. Intensive Care Med 2007, 33: 495-502; Leverve X, Mustafa I, Novak I, et al: Lactate Metabolism in Acute Uremia. J Ren Nutr 2005, 15: 58-62). Reasons for preference of bicarbonate are for clinicians a prejudice that "lactate contributes to metabolic strain" and a loss lactate as a marker of anaerobic metabolism in shock states. Bicarbonate is unstable in solution with cations and requires two chamber bags which is more demanding to produce and to store than a single chamber bag with a stable solution buffered with Na-lactate. Two chamber bag needs a special weld between the two chambers which is broken immediately before the bag is used, another option is to provide one of the components (usually bicarbonate) in a separate container. Mixing of the two components gives rise to final solution with a limited stability which is for instant application. Single chamber bags contain only stable solutions which posses longer exspiration when stored than the two chamber bags.

Besides manipulation with buffer base the exclusion of calcium is necessary to allow for usage as a dialysis fluid and also as a predilutional (i.e. applicable proximal to the filter) hemofiltration solution. The reason is a regional uptake of calcium by citrate where exogennous influx of calcium to the proximal circuit neutralises citrate, causes increase of citrate dosage and thus increases citrate systemic load. Partial neutralisation of citrate already during passage through the filter and blood set brings shorter filter survival associating with clotting at the venous (distal) end of the set (Balik M, Zakharchenko M, Otahal M, et al: Quantification of systemic dose of substrates for intermediate metabolism during citrate anticoagulation of continous renal replacement therapy. Blood Purif 2012, 33: 80-87; Balik M, Zakharchenko M, Leden P, et al: Bioenergetic gain of citrate anticoagulated continuous haemodiafiltration - comparative study of two algorithms. J Crit Care 2012, published on line 27.8., Epub ahead of print).

As it has been already suggested, citrate anticoagulation has also an impact on magnesium metabolism however, the affinity of citrate to magnesium may be less clinically important than to calcium. Recent paper show that the levels of magnesium in current available solutions are insufficient in case of citrate anticoagulation and may lead to depletion of relatively low body pool of magnesium within couple of days (Zakharchenko M, Balik M, Leden P, et al: Citrate anticoagulated continuous haemodiafiltration: Focus on ionised magnesium. Intensive Care Med 2011, 37, S336 (abstract)). Besides the elimination technique there are many other effects reducing body stores of magnesium (diuretics, intestinal losses). Important issue is that the extracellular magnesium does not well reflect a status of main body pool of intracellular magnesium. Hypomagnesemia has an impact on incidence of heart arrhythmias and myopathies (Soliman HM, Mercan D, Lobo SS, Melot C, Vincent JL: Development of ionized hypomagnesemia is associated with higher mortality rates. Crit Care Med 2003; 31: 1082-1087).

Standard fluids do not contain phosphorus because its low excretion and body cummulation is presumed with renal insufficiency. In case of CRRT in critically ill the indication comes earlier than in a classic intermittent dialysis and therefore, the levels of phosphorus are lower. Phosphorus has to be replenished very early in case of continuous modality. Eventual omission or insufficient substitution may harm a patient with severe hypophosphatemia. This is partially avoided by few of the existing solutions (Medisol Bi0, Table 1). Again, a negative impact of severe hypophosphatemia on muscles, heart, nervous system and patients nutrition was proved (Broman M, Carlsson O, Friberg H, Wieslander A, Godaly G: Phosphate-containing dialysis solution prevents hypophosphatemia during continuous renal replacement therapy. Acta Anaesthesiol Scand 2011; 55: 39-45; Demirjian S, Teo BW, Guzman JA, et al: Hypophosphatemia during continuous hemodialysis is associated with prolonged respiratory failure in patients with acute kidney injury. Nephrol Dial Transplant 2011; 26: 3508-14).

A very important aspect of CRRT is the cost of the treatment. A demand is for safe, correctly indicated modality associating with zero mortality of renal failure, at the same time the modality must be financialy sustainable. The expenses should be covered within the lump sum for an ICU day which is a way of reimbursement in most countries with developed health care systems. This means that a payment should cover filter, sets, dialysis/substitution fluids, anticoagulation and monitoring (Balik M, Zakharchenko M, Leden P, et al: Bioenergetic gain of citrate anticoagulated continuous haemodiafiltration comparative study of two algorithms. J Crit Care 2012, published on line 27.8., Epub ahead of print). Options available on current market allow for coverage of all expenses only in certain CRRT devices and only with limited flows of commercially produced fluids which are sufficient for mostly stable patients.

If a higher flow of dialysis or filtration is needed it is possible to exceed the lump sum payment and a modality becomes financially uncovered. This may happen regardless of anticoagulation regimen, i.e. with or without citrate.

### Summary of the invention

The subject of this invention is a dialysis and substitution fluid designed for elimination techniques with citrate anticoagulation in the intensive care petting, where the fluid composition comprises 130 mmol/l Na, 2 mmol/l K, 1.5 mmol/l Mg, 116 mmol/l CI, 1.0 mmol/l P, 18 mmol/l Na-lactate and 5.6 mmol/l glucose, whereby osmolality of the fluid is 274 mOsm/kg.

In practice it can comprise 125 to 135 mmol/l Na, 0 to 4 mmol/l K, 0.8 to 1.8 mmol/l Mg, 110 to 125 mmol/l CI, 0.8 to 1.2 mmol/l P, 15 to 25 mmol/l Na-lactate and 5.0 to 6.0 mmol/l glucose, whereby osmolality of the fluid is in the range of 270 to 285 mOsm/kg.

Another subject of the invention is a single chamber bag of 5000 ml volume generally applicable with citrate anticoagulation which contains above described dialysis and substitution fluid.

### Examples of preferred embodiments

The subject of this invention is a new single chamber bag, generally applicable with citrate anticoagulation even in unstable critically ill patients. The single chamber bag is equipped with connectors enabling connectivity to various devices available on market (Fresenius Multifiltrate, Aquarius Baxter, Braun Diapact, Kimal, and others). It has a volume of 5000 ml of universal content allowing for an application as dialysis or hemofiltration solution with parallely applied citrate anticoagulation. The presumed application is with 4% trisodium citrate. Its ion content and osmolality would not threaten a patient even in human error and accidental incorrect usage with another type of anticoagulation (i.e. with heparin).

The bag contains dialysis and hemofiltration solution for citrate anticoagulation, with final concentrations of 130 mmol/l Na, 2 mmol/l K, 1.5 mmol/l Mg, 116 mmol/l CI, 1.0 mmol/l P, 18 mmol/l Na-lactate and 5.6 mmol/l glucose, osmolality of the fluid is about 274 mOsm/kg. It has reduced sodium content, elevated chloride content and lowered concentration of lactate buffer. The reason is its presumed usage with 4% trisodium citrate. Hypernatremic citrate solution carries a risk of elevation of plasmatic sodium and development of metabolic alkalosis due to citrate load (vide supra). Low sodium content is accompanied by lowered concentration of lactate buffer which is partially replaced with chloride anion.

The bag contains no calcium to avoid neutralisation of citrate already at the proximal part of blood circuit and increasing a demand for citrate dosage. The absence of calcium reduces necessary dosage of citrate to the proximal blood circuit, extends its survival and reduces overall systemic load of citrate (Balik M, Zakharchenko M, Leden P, et al: Bioenergetic gain of citrate anticoagulated continuous haemodiafiltration - comparative study of two algorithms. J Crit Care 2012, published 27.8., Epub ahead of print).

The buffering with sodium salt of lactate is chosen due to the absence of evidence in favour of the bicarbonate buffer, tested outstanding tolerance of lactate in critically ill (Balik M, Zakharchenko M, Otahal M, et al: Quantification of systemic dose of substrates for intermediate metabolism during citrate anticoagulation of continous renal replacement therapy. Blood Purif 2012, 33: 80-87), stability of lactate buffered fluids and its lower costs. A dosage of lactate buffer is chosen at the lower limit of acceptable range because eventual pH and base excess (BE) decreases may be corrected by increasing blood flow and associated dosage of citrate which adjusts pH and BE upwards. After patients improvement (in many patients CRRT is commenced in acidosis) the blood pump may be slowed down and the associated citrate dosage reduced. A low level of lactate should not lead to significantly elevated blood levels and arterial lactate may remain a marker on anaerobic metabolism. Systemic dosage of lactate should be lower than in "classic" lactate buffered CRRT. The expected systemic load is about 30-40 mmol/h, i.e. a daily gain of 750-1000 mmol of lactate, which is well metabolized dose even in haemodynamically unstable patient or in severe liver insufficiency (Levy B, Mansart A, Montemont C, et al: Myocardial lactate deprivation is associated with decreased cardiovascular performance, decreased myocardial energetics, and early death in endotoxic shock. Intensive Care Med 2007, 33: 495-502; Leverve X, Mustafa I, Novak I, et al: Lactate Metabolism in Acute Uremia. J Ren Nutr 2005, 15: 58-62). A stability of lactate fluid allows for usage of a single chamber bag which further reduces its price.

In comparison with other solutions this bag has normal level of magnesium which simplifies its monitoring and avoids development of hypomagnesemia while on CRRT. The set level should compensate for daily losses of 20-30 mmol (Zakharchenko M, Balik M, Leden P, et al: Citrate anticoagulated continuous haemodiafiltration: Focus on ionised magnesium. Intensive Care Med 2011, 37, S336 (abstract)) caused both by citrate anticoagulation and the elimination technique without magnesium substitution. Besides magnesium there is also a normal level of phosphorus limiting a decrease of phosphate to sometimes reported severely low levels associating with clinical impairment (Broman M, Carlsson O, Friberg H, Wieslander A, Godaly G: , Phosphate-containing dialysis solution prevents hypophosphatemia during continuous renal replacement therapy, Acta Anaesthesiol Scand 2011; 55: 39-45; Demirjian S, Teo BW, Guzman JA, et al: Hypophosphatemia during continuous hemodialysis is associated with prolonged respiratory failure in patients with acute kidney injury. Nephrol Dial Transplant 2011; 26: 3508-14).

A standard level of glucose is set as in other solutions in the range enabling safe and tight glucose control according to the current recommendations (Van den Berghe G, Wilmer A, Hermans G, et al: Intensive insulin therapy in the medical ICU. N Engl J Med 2006, 354: 449-61). The level of potassium is set at 2.0 mmol/l which allows for flexible adjustments and it makes the solution useful even in severe hyperkalemia.

The content and stability of the solution allows for usage of a single chamber bag with presumed longer exspiration period than in the bicarbonate buffered two chamber bags. Application of a single chamber bag associates with its lower production cost which should be reflected in market price approaching a price of currently available common lactate buffered fluids. The use of polypropylene (established system of GML company) eliminates a need of another transport wrapping on an individual bag. The volume of a single bag is a standard 5000 ml to enable its usage on current devices for elimination techniques and their weight system which is typically calibrated for weights around this level.

The expected application is with a turnover of dialysate/filtrate of 20-35 ml/kg.h, i.e. about 1500-4000 ml/h. With regards to the evidence based medicine the usual dialysis/filtration dosage is between 1500-3000 ml/h. Even in continuous venovenous hemofiltration (CWH) and division of substitution to 50 % predilution (i.e. administration prefilter) and 50 % postdilution (i.e. given postfilter) a blood flow of 100-150 ml/min would be satisfactory to keep the filtration fraction on the filter under 20%. This is satisfactory and significantly higher blood flow than current recommendations for an application of 4% trisodium citrate (80-110 ml/min, see Morgera S, Haase M, Ruckert M, et al: Regional Citrate Anticoagulation in Continuous Hemodialysis - Acid-Base and Electrolyte Balance at an Increased Dose of Dialysis. Nephron Clin Pract 2005, 101: 211-219; Morgera S, Schneider M; Slowinski T, et al: A safe citrate anticoagulation protocol with variable treatment efficacy and excellent control of the acid-base status. Crit Care Med 2009, 37: 2018-2024) which also allows for higher flexibility in patient's ultrafiltration. As explained above a dose of citrate prefilter is related to blood flow. If calcium free fluids are applied the expected consumption of 4% trisodium citrate is between 170-220 ml/h. According to the published papers this would lead to a citrate systemic load between 14 and 18 mmol/h (Balik M, Zakharchenko M, Leden P, et al: Bioenergetic gain of citrate anticoagulated continuous haemodiafiltration - comparative study of two algorithms. J Crit Care 2012, published on line 27.8, Epub ahead of print). The estimated cost saving compared to a standard bicarbonate buffered bags is (calculation as of December 2011) approximately 80-100 CZk (3.3-4.2 EUR) per one hour on CRRT, this is approximately about 2100 CZK or 90 EUR per day on CRRT. Calculating average length of CRRT between 7 to 14 days makes about 15.000 to 30.000 CZk or 630-1260 EUR/1 patient. A similar dialysis/substitution fluid currently does not exist in a company portfolio of products worldwide. That means simply manufactured, long term stable, single chamber and relatively cheap bag which is generally applicable with citrate anticoagulated CRRT.

## Claims

1. Dialysis and substitution fluid, designed for elimination techniques with citrate anticoagulation in the intensive care setting, **characterized in that** the fluid comprises 130 mmol/l Na, 2 mmol/l K, 1.5 mmol/l Mg, 116 mmol/l CI, 1.0 mmol/l P, 18 mmol/l Na-lactate and 5.6 mmol/l glucose, where osmolality of the fluid is 274 mOsm/kg.

2. Single chamber bag generally applicable with citrate anticoagulation, **characterized in that** it contains dialysis and substitution fluid according to claim 1.

3. Single chamber bag according to claim 2, **characterized in that** it has a volume of 5000 ml.

## Patentansprüche

1. Dialyse- und Substitutionsflüssigkeit, bestimmt für Eliminationstechniken mit Citrat-Antikoagulation im Intensivpflegebereich, **dadurch gekennzeichnet, dass** die Flüssigkeit 130 mmol/l Na, 2 mmol/l K, 1,5 mmol/l Mg, 116 mmol/l CI, 1,0 mmol/l P, 18 mmol/l Na-Laktat und 5,6 mmol/l Glukose aufweist, wobei eine Osmolalität der Flüssigkeit 274 mOsm/kg beträgt.

2. Einkammerbeutel, grundsätzlich anwendbar mit einer Citrat-Antikoagulation, **dadurch gekennzeichnet, dass** er eine Dialyse- und Substitutionsflüssigkeit nach Anspruch 1 enthält.

3. Einkammerbeutel nach Anspruch 2, **dadurch gekennzeichnet, dass** er ein Volumen von 5.000 ml hat.

## Revendications

1. Liquide de dialyse et de substitution, conçu pour des techniques d'élimination avec anticoagulation au citrate dans le cadre de soins intensifs, **caractérisé en ce que** le liquide comprend 130 mmol/L de Na, 2 mmol/L de K, 1,5 mmol/L de Mg, 116 mmol/L de Cl, 1,0 mmol/L de P, 18 mmol/L de lactate de Na et 5,6 mmol/L de glucose, où l'osmolalité du liquide étant de 274 mOsm/kg.

2. Poche à chambre simple généralement utilisable avec anticoagulation au citrate, **caractérisée en ce qu'**elle contient du liquide de dialyse et de substitution selon la revendication 1.

3. Poche à chambre simple selon la revendication 2, **caractérisée en ce qu'**elle a un volume de 5000 mL.
